(19) Europäisches Patentamt
European Patent Office
Office européen des brevets

(11) Veröffentlichungsnummer: **0 178 611 B1**

(12) # EUROPÄISCHE PATENTSCHRIFT

(45) Veröffentlichungstag der Patentschrift: **17.06.92**

(51) Int. Cl.$^5$: **C07D 251/70**

(21) Anmeldenummer: **85112973.4**

(22) Anmeldetag: **12.10.85**

Die Akte enthält technische Angaben, die nach dem Eingang der Anmeldung eingereicht wurden und die nicht in dieser Patentschrift enthalten sind.

(54) **Verfahren zur Herstellung von N,N',N"-Tris(2-hydroxypropyl)melamin.**

(30) Priorität: **19.10.84 DE 3438387**

(43) Veröffentlichungstag der Anmeldung:
**23.04.86 Patentblatt 86/17**

(45) Bekanntmachung des Hinweises auf die Patenterteilung:
**17.06.92 Patentblatt 92/25**

(84) Benannte Vertragsstaaten:
**CH DE GB LI**

(56) Entgegenhaltungen:
**EP-A- 0 051 753**
**US-A- 3 244 713**

(73) Patentinhaber: **BASF Aktiengesellschaft**
**Carl-Bosch-Strasse 38**
**W-6700 Ludwigshafen(DE)**

(72) Erfinder: **Ebel, Klaus, Dr.**
**Ungsteiner Strasse 18**
**W-6700 Ludwigshafen(DE)**
Erfinder: **Reuther, Wolfgang, Dr.**
**Am Pferchelhang 16**
**W-6900 Heidelberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 178 611 B1

## Beschreibung

Die vorliegende Erfindung betrifft ein neues Verfahren zur Herstellung von N,N',N''-Tris(2-hydroxypropyl)melamin, durch Umsetzung von Melamin mit einem Überschuß an Isopropanolamin.

Aus der US-A-3, 244, 713 ist die Umsetzung von Isopropanolamin zu Melamin im Molverhältnis von 5:1 bekannt. DIe Ergebnisse bzw. der Nebenproduktbildung lassen zu wünschen übrig.

Aus der US-A-4 312 988 ist die Umsetzung von Melamin mit Ethanolamin bzw. mit Isopropanolamin bekannt. Dort wurde gefunden, daß bei der Reaktion von Melamin mit Ethanolamin anstelle von N,N',N''-Tris(2-hydroxyethyl)melamin in einer Konkurrenzreaktion unter Wasserabspaltung hauptsächlich Isomelamine gebildet werden, die für die geringe Ausbeute an N,N',N''-Tris(2-hydroxyethyl)melamin verantwortlich sind. Beispielsweise wurden bei der Umsetzung von Ethanolamin mit Melamin im Molverhältnis 3,2:1 bei 82 % Umsatz 20 % an Isomelaminen, bei 95 % Umsatz bereits 50 % an Isomelaminen und bei 99 % Umsatz schließlich 100 % an Isomelaminen erhalten.

Es wurde daher vorgeschlagen, anstelle von Ethanolamin Isopropanolamin zu verwenden. Nach der Lehre der US-A-4 312 988 sollte nämlich die Isomelaminbildung drastisch reduziert werden können, wenn man das "geradkettige" Ethanolamin durch ein "verzweigtes" Isoalkanolamin ersetzt.

Aus dem dort beschriebenen Beispiel, in dem Isopropanolamin und Melamin im Molverhältnis 3,2:1 umgesetzt werden, geht aber hervor, daß man dabei ein Endprodukt erhält, dessen Anteil an den unerwünschten Isomelaminen immer noch 40 % beträgt.

Eine Anregung für die Herstellung von N,N',N''-Tris(2-hydroxyethyl)melamin, das annähernd frei von Isomelaminen ist und bei der man von Melamin und Isopropanolamin ausgeht, konnte aus diesem Vorschlag nicht entnommen werden.

Die Darstellung von reinem, kristallinem N,N',N''-Tris(2-hydroxypropyl)melamin gelang nämlich bisher nur durch die Umsetzung von kostspieligem Cyanurchlorid mit Isopropanolamin, wobei bei dieser Reaktion außerdem eine große Menge an Salz anfällt (J. Am. Chem. Soc. 73, 2985 (1951)).

Aufgabe der vorliegenden Erfindung war es deshalb, ein Verfahren zur Herstellung von N,N',N''-Tris(2-hydroxypropyl)melamin, ausgehend von Melamin und Isopropanolamin, bereitzustellen, in dem die Isomelaminbidlung verhindert wird.

Es wurde nun gefunden, daß die Herstellung von N,N',N''-Tris(2-hydroxypropyl)melamin durch Umsetzung von Melamin mit Isopropanolamin in Gegenwart von sauren Katalysatoren vorteilhaft gelingt, wenn man die Reaktion bei einer Temperatur von 120 bis 300 °C und einem Überschuß an Isopropanolamin durchführt und dabei ein Molverhältnis Von freiem Isopropanolamin : Summe aus freiem und umgesetztem Melamin von 4:1 während der gesamten Reaktionszeit nicht unterschreitet.

N,N' ,N''-Tris(2-hydroxypropyl)melamin besitzt folgende Strukturformel:

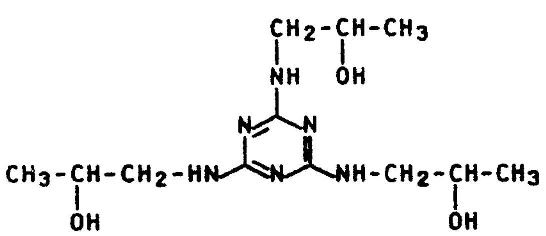

Das erfindungsgemäße Verfahren wird zweckmäßigerweise so durchgeführt, daß man ein Gemisch aus Melamin, Isopropanolamin, saurem Katalysator und gegebenenfalls einem Lösungsmittel vorlegt und unter Rühren auf eine Temperatur von 120 bis 300 °C, insbesondere von 150 bis 180 °C erhitzt.

Man arbeitet im allgemeinen bei atmosphärischem Druck, um jedoch den oberen Temperaturbereich (180 bis 300 °C) zu erreichen, muß ein Druck von 1 bis 25 bar eingehalten werden.

Weiterhin empfiehlt es sich, die Umsetzung in Gegenwart eines Schutzgases durchzuführen. Das Schutzgas wird dabei im allgemeinen über die Oberfläche der Reaktionsmischung geleitet. Geeignete Schutzgase sind z.B. Edelgase und insbesondere Stickstoff.

Als saure Katalysatoren kommen alle starken und mittelstarken Säuren in Betracht, z.B. Flußsäure, Salzsäure. Bromwasserstoffsäure, Schwefelsäure, Salpetersäure Phosphorsäure, Amidosulfonsäure, Thiocyansäure, p-Toluolsulfonsäure oder Methansulfonsäure.

Die Sauren können entweder in freier Form oder als Melamin- oder Isopropanolaminsalz zugegeben werden. Weiterhin ist auch die Zugabe als Salz einer Base, die schwächer als Isopropanolamin ist, möglich,

beispielsweise als Ammoniumsalz.

Anstelle der genannten Protonsäuren katalysieren auch Lewis-Säuren, wie Bortrifluorid, Aluminiumchlorid, Zink(IV)chlorid, Antimon(V)fluorid oder Eisen(III)bromid die Reaktion.

Pro Mol Melamin werden 0.05 bis 3 Mol, vorzugsweise 0.1 bis 1 Mol Katalysator eingesetzt. Für den Fall, daß man die Protonsäure in Form ihres Melaminsalzes verwendet, muß die Melaminmenge aus dem Salz berücksichtigt werden. Das erforderliche Molverhältnis freies Isopropanolamin : Summe aus freiem und umgesetztem Melamin von 4:1 darf nicht unterschritten werden. Mit zunehmender Katalysatormenge ist ein Anstieg der Reaktionsgeschwindigkeit zu beobachten.

Das erfindungsgemäße Verfahren wird vorzugsweise in Gegenwart von organischen Lösungsmitteln durchgeführt, jedoch ist es auch möglich in deren Abwesenheit zu arbeiten.

Als organische Lösungsmittel kommen Polyole in Betracht, beispielsweise Ethylenglykol, 1,2-Propylenglykol, Diethylenglykol oder Triethylenglykol. Die Verwendung von Ethylenglykol ist bevorzugt.

Ein entscheidener Verfahrensparameter ist das Molverhältnis freies Isopropanolamin : Summe aus freiem und umgesetztem Melamin, das während der gesamten Reaktionszeit den Wert 4:1 nicht unterschreiten darf. Als umgesetztes Melamin werden dabei N,N' ,N''-Tris(2-hydroxypropyl)amin sowie falls vorhanden, dessen Vorstufen N-Mono(2-hydroxypropyl)melamin und N,N'-Bis(2-hydroxypropyl)melamin betrachtet.

Eine bevorzugte Ausführungsform des erfindungsgemäßen Verfahrens besteht darin, daß man während der gesamten Reaktionszeit ein Molverhältnis freies Isopropanolamin : Summe aus freiem und umgesetztem Melamin von 5:1 bis 12:1 einhält. Die Anwendung eines Molverhältnisses >15:1 bringt keine Vorteile mehr, da sich in diesem Falle die Reaktionsdauer beträchtlich erhöht.

Zur Isolierung des Wertproduktes neutralisiert man die jeweilige Katalysatorsäure, indem man eine Base, z.B. Natriumhydroxid, Kaliumhydroxid, Natriumcarbonat, Kaliumcarbonat, Calciumcarbonat oder Bariumcarbonat zugibt. Dann wird überschüssiges Isopropanolamin und das gegebenenfalls mitverwendete organische Lösungsmittel unter vermindertem Druck (ca. 30 mbar) bei einer Temperatur von 150 bis 200 °C abdestilliert und der resultierende Rückstand mit Wasser versetzt.

Dabei bilden sich zwei Phasen, eine organische und eine wäßrige Phase, die die Salze enthält. Man trennt die organische Phase ab und versetzt sie erneut mit Wasser. Beim Abkühlen kristallisert dann reines N,N' ,N''-Tris(2-hydroxypropyl)melamin in Form farbloser Kristalle aus; diese werden abgetrennt und getrocknet.

Mittels des erfindungsgemäßen Verfahrens erhält man nahezu isomelaminfreies N,N' ,N''-Tris(2-hydroxypropyl)melamin, wodurch gewährleistet ist, daß dessen volle NH- und OH-Funktionalität erhalten bleibt. Das Zielprodukt fällt in hoher Reinheit und guter Ausbeute an.

N,N' ,N''-Tris(2-hydroxypropyl)melamin ist ein wertvolles Zwischenprodukt, beispielsweise für die Herstellung von Urethanen.

Die nachfolgenden Beispiele sollen die Erfindung näher erläutern.

Beispiele 1 bis 6

Die Beispiele 1 bis 6 wurden unter den selben Reaktionsbedingungen lediglich mit unterschiedlichem Isopropanolaminüberschuß durchgeführt. Als saurer Katalysator diente jeweils Ammoniumchlorid.

Durchführung der Beispiele 1 bis 6: Melamin, Katalysator, Isopropanolamin und Ethylenglykol als Lösungsmittel wurden unter Rühren und Überleiten eines schwachen Stickstoffstroms in einem 200 °C heißen Ölbad unter Rückfluß gerührt. Die Reaktion wurde durch Probenentnahme mittels Hochdruckflüssigkeitschromatographie (HPLC) verfolgt.

Die Einwaagen und die HPLC-Auswertung sind in der nachfolgenden Tabelle aufgeführt. Es wurde jeweils die Nebenproduktmenge bei möglichst hohem Anteil an N,N' ,N''-Tris(2-hydroxypropyl)melamin verglichen. Dabei erkennt man deutlich, wie die Nebenproduktmenge mit zunehmendem Isopropanolaminüberschuß abnimmt.

Lediglich in Beispiel 6 (Molverhältnis Isopropanolamin : Melamin 15:1) wurde die Reaktion wegen der extrem langen Reaktionszeit von über 50 Stunden vorzeitig abgebrochen.

**Tabelle**

| | Beispiel 1 | | Beispiel 2 | | Beispiel 3 | | Beispiel 4 | | Beispiel 5 | | Beispiel 6 | |
|---|---|---|---|---|---|---|---|---|---|---|---|---|
| | Einwaage [g] | Molverhältnis | Einwaage [g] | Molverhältnis | Einwaage [g] | Molverhältnis | Einwaage [g] | Molverhältnis | Einwaage [g] | Molverhältnis | Einwaage [g] | Molverhältnis |
| Melamin | 31,5 | 1 | 31,5 | 1 | 31,5 | 1 | 31,5 | 1 | 25,2 | 1 | 18 | 1 |
| Ethylenglykol | 31,0 | 2 | 31,0 | 2 | 46,5 | 3 | 46,5 | 3 | 24,8 | 2 | 17,7 | 2 |
| Ammoniumchlorid | 13,4 | 1 | 13,4 | 1 | 13,4 | 1 | 13,4 | 1 | 10,7 | 1 | 7,6 | 1 |
| Isopropanolamin | 93,8 | 5 | 112,5 | 6 | 131,3 | 7 | 150,0 | 8 | 150,0 | 10 | 160,7 | 15 |

| | HPLC-Auswertung [Flächen %] | HPLC-Auswertung [Flächen %] | HPLC-Auswertung [Flächen %] | HPLC-Auswertung [Flächen %] | HPLC-Auswertung [Flächen %] | HPLC-Auswertung [Flächen %] |
|---|---|---|---|---|---|---|
| Bis-HPM* | 3 | 2 | 2 | 6 | 4 | 16 |
| Tris-HPM** | 32 | 57 | 71 | 79 | 83 | 80 |
| Nebenprodukte | 65 | 41 | 27 | 15 | 11 | 5 |

* Bis-HPM = N,N'-Bis(2-hydroxypropyl)melamin

** Tris-HPM = N,N',N''-Tris(2-hydroxypropyl)melamin

## Beispiel 7

Man rührte eine Mischung aus 504 g (4 Mol) Melamin, 214 g (4 Mol) Ammoniumchlorid und 2400 g (32 Mol) Isopropanolamin in 496 g (8 Mol) Ethylenglykol bei einer Temperatur von 162 bis 178 °C 63 Stunden

unter Rückfluß, wobei ein schwacher Stickstoffstrom über die Oberfläche der Reaktionsmischung geleitet wurde. Anschließend wurde auf eine Temperatur von 90 °C abgeküht und das Reaktionsgemisch durch Zugabe von 320 g (4 Mol) 50 gew.%iger Natronlauge neutralisiert. Daraufhin destillierte man überschüssiges Isopropanolamin sowie Ethylenglykol bei einem Druck von 30 mbar und bei einer Sumpftemperatur von bis zu 200 °C ab.

Der Rückstand wurde mit 800 g warmem Wasser versetzt, worauf sich zwei Phasen bildeten. Die wäßrige Phase wurde abgetrennt und die organische Phase erneut mit 200 ml Wasser versetzt, wobei sich eine homogene Lösung bildete. Beim Abkühlen kristalliserte N,N' ,N''-Tris(2-hydroxypropyl)melaminaus und wurde abgesaugt und getrocknet. Man erhielt 850 g (71 %) farblose Kristalle von Schmp. 131 °C.

Nach dem Einengen der Mutterlauge auf ca. ein Drittel ihres ursprünglichen Volumens erhielt man weitere 197 g (17 %) N,N' ,N''-Tris(2-hydroxypropyl)melamin vom Schmp. 126 °C.

Das erhaltene N,N' ,N''-Tris(2-hydroxypropyl)melamin ist gemäß analytischer Untersuchung mittels HPLC mit einer aus Cyanurchlorid und Isopropanolamin hergestellten Vergleichsprobe identisch und besitzt einen Reinheitsgrad von 99 %. Die spektroskopischen Daten ($^1$H-NMR-Spektrum, IR-Spektrum und Massenspektrum) bestätigen ebenfalls die Struktur.

## Patentansprüche

1. Verfahren zur Herstellung von N,N' ,N''-Tris(2-hydroxypropyl)melamin durch Umsetzung von Melamin mit Isopropanolamin in Gegenwart von sauren Katalysatoren, dadurch gekennzeichnet, daß man die Reaktion bei einer Temperatur von 120 bis 300 °C und einem Überschuß an Isopropanolamin durchführt und dabei ein Molverhältnis von freiem Isopropanolamin : Summe aus freiem und umgesetztem Melamin von 4:1 während der gesamten Reaktionszeit nicht unterschreitet.

2. Verfahren gemäß Anspruch 1, dadurch gekennzeichnet, daß man die Umsetzung bei einer Temperatur von 150 bis 180 °C und bei atmosphärischem Druck durchführt.

3. Verfahren gemäß Anspruch 1, dadurch gekennzeichnet, daß man die Umsetzung in Gegenwart eines organischen Lösungsmittels durchführt.

4. Verfahren gemäß Anspruch 1, dadurch gekennzeichnet, daß man die Umsetzung in Gegenwart von Ethylenglykol als organischem Lösungsmittel durchführt.

5. Verfahren gemäß Anspruch 1, dadurch gekennzeichnet, daß man während der gesamten Reaktionszeit ein Molverhältnis freies Isopropanolamin : Summe aus freiem und umgesetztem Melamin von 5:1 bis 12:1 einhält.

## Claims

1. A process for the preparation of N,N',N''-tris-(2-hydroxypropyl)-melamine by reacting melamine with isopropanolamine in the presence of an acidic catalyst, wherein the reaction is carried out at from 120 to 300 °C and using an excess of isopropanolamine, and a molar ratio of free isopropanolamine to the sum of free and converted melamine does not fall below 4:1 during the entire reaction time.

2. A process as claimed in claim 1, wherein the reaction is carried out at from 150 to 180 °C and under atmospheric pressure.

3. A process as claimed in claim 1, wherein the reaction is carried out in the presence of an organic solvent.

4. A process as claimed in claim 1, wherein the reaction is carried out in the presence of ethylene glycol as the organic solvent.

5. A process as claimed in claim 1, wherein the molar ratio of free isopropanolamine to the sum of free and converted melamine is maintained at from 5:1 to 12:1 during the entire reaction time.

## Revendications

**1.** Procédé de préparation de la N,N',N''-tris(2-hydroxypropyl)mélamine par réaction de la mélamine avec l'isopropanolamine en présence de catalyseurs acides, caractérisé en ce que l'an effectue la réaction à une température de 120 à 300 ° C en présence d'un excès d'isopropanolamine qui soit telle que, pendant tout le temps de la réaction, le rapport molaire de l'isopropanolamine libre à la somme de la mélamine libre et de la mélamine ayant réagi ne tombe pas au-dessous de 4:1.

**2.** Procédé suivant la revendication 1, caractérisé en ce que l'on effectue la réaction à une température de 150 à 180 ° C et sous la pression atmosphérique.

**3.** Procédé suivant la revendication 1, caractérisé en ce que l'on effectue la réaction en présence d'un solvant organique.

**4.** Procédé suivant la revendication 1, caractérisé en ce que l'on effectue la réaction en présence d'éthylèneglycol comme solvant organique.

**5.** Procédé suivant la revendication 1, caractérisé en ce que l'on maintient, pendant tout le temps de la réaction, un rapport molaire de l'isopropanolamine libre à la somme de la mélamine libre et de la mélamine ayant réagi de 5:1 à 12:1.